# EUROPEAN PATENT SPECIFICATION

(11) **EP 3 347 710 B1**
(45) Date of publication and mention of the grant of the patent: **01.11.2023**
(21) Application number: 16766911.8
(22) Date of filing: 13.09.2016
(51) Int. Cl.: G01N 27/327, G01N 33/487

(54) **USE OF HANDELD APPARATUS, DISPOSABLE TEST UNIT AND HANDHELD TEST METER FOR TESTING A SAMPLE OF BODY FLUID**
VERWENDUNG EINER TRAGBAREN VORRICHTUNG, EINER EINWEGTESTEINHEIT UND EINES TRAGBAREN TESTMESSGERÄTS ZUM TESTEN EINER KÖRPERFLÜSSIGKEITSPROBE
UTILISATION D'UN APPAREIL PORTATIF, UNITÉ DE TEST JETABLE ET APPAREIL DE MESURE DE TEST PORTATIF POUR TESTER UN ÉCHANTILLON DE FLUIDE CORPOREL

(30) Priority: 13.09.2015 DK 201500541
(43) Date of publication of application: 18.07.2018
(73) Proprietor: Pro-ino Development APS, 7400 Herning (DK)
(72) Inventor: HANSEN, Jens, 9330 Dronninglund (DK)
(74) Representative: Larsen & Birkeholm A/S
(86) International application number: PCT/EP2016/071547
(87) International publication number: WO 2017/042398

(56) References cited:
- WO-A1-2014/072753
- DE-A1-102006 045 952
- US-A1- 2002 084 184
- US-A1- 2009 223 287
- US-A1- 2010 015 006
- US-A1- 2011 102 152
- US-A1- 2015 083 609
- THADDAEUS A. WEBSTER ET AL: "Electrochemical detection of Pseudomonas aeruginosa in human fluid samples via pyocyanin", BIOSENSORS AND BIOELECTRONICS, vol. 60, 29 April 2014 (2014-04-29), pages 265-270, XP055289491, NL ISSN: 0956-5663, DOI: 10.1016/j.bios.2014.04.028

## Description

### Technical field of the invention

The present invention relates to the use of handheld apparatuses, disposable electrochemical test strips and handheld test meters for testing a sample of body fluid.

### Background of the invention

Based on age, gender, genetic background, and lifestyle, people generally have health problems and chronic illnesses at different points in their lives. Knowledge about an individual person's physiological and/or medical state is key for both the individual self and to his/her health care provider in order to identify issues before they start, and to prevent them from occurring. Regular health exams and tests can help find problems before they start. However, only few people attend regular checkups with his/her health care provider. This is due to several factors, such as opening hours at the health care provider, and consultancy costs. Furthermore, some people are only interested in monitoring their physiological state, e.g. to analyze if specific exercise and change in eating habits has an effect - without involving his/her health care provider.

Especially when it comes to sexually transmitted diseases including chlamydia, gonorrhea, and herpes, people do not like such information to be a part of their medical record.

It is therefore desirable to provide an easy-to-use method that can give an individual information about his/her physiological and/or medical state. Self-testing of an individual's body fluids, such as blood or urine, is not a new concept. However, most existing self-tests only provide the opportunity of one type of test. Hence, there is a need for a system capable of handling multiple types of tests.

US20110102152 discloses a double side identification notation-containing test strip and a test instrument therefor. The test strip comprises a substrate, having a first surface and a second surface, and at least a test area and an identification area provided thereon, hereby forming a double side identification notation-containing test strip. In said double side identification notation-containing test strip, a test area and an identification area are disposed respectively on different surfaces, thus increasing the area of said identification area and sets of digital identification electrodes, and when it is operated in cooperation with a resistor, it generates a plurality sets of analog identification signals.

DE 10 2006 045 952 A1 discloses a biosensor with a test strip and a meter. A passive RFID tag is arranged on the test strip. A RFID-reader is arranged inside the meter for receiving of the radio frequency signals of the RFID-chip. The received RF signals are sent to a micro processing unit for releasing the calibration operation at the meter.

Similarly US 2009/223287 A1 discloses a bio-monitoring system where a RFID tag is embedded in a test strip and a RFID reader is embedded in a test meter.

### Summary of the invention

A first aspect relates to the use of a handheld apparatus as defined in claim 1 configured for testing a sample of body fluid.

A second aspect relates to the use of a disposable electrochemical test unit as defined in claim 4 for testing a sample of body fluid.

A third aspect relates to the use of a handheld test meter as defined in claim 8 for testing a sample of body fluid.

### Detailed description of the invention

A first aspect relates to the use of a handheld apparatus as defined in claim 1 for testing a sample of body fluid.

Disclosed herein is a handheld apparatus for use in testing a sample of body fluid for redox active substances. The apparatus comprises a handheld test meter adapted for receiving a disposable electrochemical test unit.

A redox active substance is a molecule that can cycle between reduced and oxidized states. The redox active substance may be a siderophore secreted by a fungus or bacterium, for example, a siderophore described by Hider and Kong (R. C. Hider and X. Kong, 2010, Nat. Prod. Rep. 27: 637-657). The siderophore may be ferrichrome, Desferoxamine B, Deferoxamine, Desferoxamine E, fusarinine C, ornibactin, rhodotorulic acid, enterobactin, bacillibactin, vibriobactin, azotobactin, pyoverdine, yersiniabactin, or pyocyanin.

The handheld apparatus also comprises a disposable electrochemical test unit adapted for electrochemical sensing of a redox active substance. The handheld test meter works in conjunction with the disposable electrochemical test unit that comprises at least two electrodes (working and counter electrodes) disposed within the receptacle.

The handheld test meter contains the electronics for applying the potential between the working and counter electrodes in the disposable electrochemical test unit, observing the current, and generating a determination of a redox active substance and conveying it to the user. An indication of the presence or absence of detectable amounts of a redox active substance in the sample is generated based on the observed current. Detection of a redox active substance in a sample of body fluid refers to the qualitative, semi-quantitative or quantitative determination of whether a redox active substance is present in a sample at detectable levels. Qualitative analysis provides merely a yes/no answer as to whether there is detection. Semi-quantitative provides an indication of amount, but with high granularity, for example as presented through a series of lights where the number of lights illuminated indicates the range into which a value falls. Quantitative analysis provides a numerical value for the amount of a redox active substance in the measured sample.

In order to handle different types of electrochemical tests, the handheld test meter is configured to adjust to such situations by running a specific application for each specific electrochemical test. The information about which specific electrochemical test application there is to be run is provided in the disposable electrochemical test unit by an electronic identification tag embedded therein.

The electronic identification tag is a passive RFID tag (Radio Frequency Identification), preferably a passive NFC (Near Field Communication) tag.

Passive RFID tags, such as a passive NFC tag, do not have their own power source. Instead, they are powered by the electromagnetic energy transmitted from the RFID reader. Because the radio waves must be strong enough to power the tags, passive RFID tags have a read range from near contact and up to 25 meters. For the present application, it is preferred that the RFID tags are configured to have a read range from direct contact (0 mm) up to a few millimeters, such as within the range of 0-2 mm. This is to avoid confusion when several disposable electrochemical test units are in close proximity of the handheld apparatus. The NFC technology is suitable for such use.

In one or more embodiments, the RFID tag is configured to have a read range within the range of 0-20 mm, such as within the range of 1-15 mm, preferably within the range of 0-5 mm.

In order to secure the correct position between the electronic identification tag and its reader, the electronic identification tag is embedded in the connector.

The handheld test meter comprises an insertion hole comprising a clamping section; and the connector comprises an engagement groove adapted to be engaged with an engagement member in the clamping section. In one or more embodiments, the engagement member is an engagement click member. The sound of a "click" reassures the user that the disposable electrochemical test unit is correctly positioned within the insertion hole of the handheld test meter.

The electronic identification tag is embedded in the engagement groove, and the engagement member comprises an electronic identification tag reader.

In one or more embodiments, the handheld test meter comprises an insertion hole comprising a clamping section; and the connector comprises an engagement member adapted to be engaged with an engagement groove in the clamping section. In one or more embodiments, the engagement member is an engagement click member. The sound of a "click" reassures the user that the disposable electrochemical test unit is correctly positioned within the insertion hole of the handheld test meter.

In one or more embodiments, the electronic identification tag is embedded in the engagement groove, and the engagement member comprises an electronic identification tag reader.

Because it can be very important to know the presence and/or concentration of a redox active substance in body fluid, a handheld apparatus has been developed by the inventor to enable the average person to sample and test a body fluid product for determining its redox active substance concentration at any given time.

In one or more embodiments, the disposable electrochemical test unit comprises a receptacle (sample receiving chamber) for a sample of body fluid.

In one or more embodiments, the disposable electrochemical test unit also comprises a reagent composition disposed in the receptacle. The reagent composition may in one or more embodiments comprise an active redox enzyme effective to oxidize or reduce the redox active substance. A "redox enzyme" refers to an enzyme that catalyzes oxidation or reduction of a redox active substance to be determined. Exemplary redox enzymes are oxidases such as glucose oxidase, and dehydrogenases such as glucose dehydrogenase or lactate dehydrogenase. The redox enzyme has an active form prior to interaction with the redox active substance, and an inactive form. In the case of an oxidase, for example, the active form is oxidized and the inactive form is reduced. As will be apparent, the specific enzyme is selected based on its specificity for the redox active substance to be analyzed. Non-limiting examples of suitable enzymes include glucose oxidase, fructose dehydrogenase, glucose dehydrogenase, alcohol oxidase, lactate oxidase, cholesterol oxidase, xanthine oxidase, and amino acid oxidases.

In one or more embodiments, the reagent composition disposed in the receptacle comprises an electron transfer reagent. An "electron transfer reagent" refers to a compound other than a redox enzyme that receives and donates electrons to and/or from another chemical species or to and/or from an electrode.

In one or more embodiments, the reagent composition disposed in the receptacle comprises a mediator compound. A "mediator compound" refers to an electron transfer reagent that in use reacts with the inactive enzyme to regenerate active enzyme. The mediator compound may also transfer electrons to or from the electrodes although this is not required.

In one or more embodiments, the reagent composition disposed in the receptacle comprises a shuttle compound. A "shuttle compound" refers to an electron transfer reagent that in use transfers electrons to and from the electrodes. In some embodiments of the invention, the shuttle is reduced by transfer of an electron from one electrode and oxidized by transfer of an electron to the other electrode. In other embodiments, the shuttle can also be reduced (or oxidized) by transfer of an electron from the reduced mediator (or to the oxidized mediator). In this instance, in one preferred embodiment, the mediator itself does not react at the electrodes.

In some embodiments, the mediator compound does not transfer electrons directly to or from the shuttle compound. In other embodiments, electron transfer between mediator and shuttle in solution can happen and in other embodiments electron transfer between mediator and shuttle is the most significant method of transferring electrons from the mediator.

The redox active substance current generated by the redox processes is detected by the handheld test meter and converted into a redox active substance concentration reading using an algorithm that relates the test current to a redox active substance concentration via a simple mathematical formula known within the art of electrochemical testing.

In one or more embodiments, the disposable electrochemical test unit comprises a diluent and/or solvent reservoir in liquid communication with the receptacle. This is an advantage when the sample of body fluid is too viscous/thick for the reagent composition to mix with the sample of body fluid; or for the situation where the sample of body fluid needs to be solubilized prior to testing, e.g., mucus.

In one or more embodiments, the diluent and/or solvent reservoir is configured as a foil sealed reservoir, a squeeze to burst packet, a peel apart welded compartment, or a squeeze to burst line sealed compartment.

In one or more embodiments, the disposable electrochemical test unit comprises a receptacle for a sample of body fluid, and a diluent and/or solvent reservoir in liquid communication with the receptacle.

In one or more embodiments, the receptacle is configured as a scoop, corer, spoon or pipette. This is an advantage for better sampling of the body fluid.

A second aspect relates to the use of a disposable electrochemical test unit as defined in claim 4 for testing a sample of body fluid.

A third aspect relates to the use of a handheld test meter as defined in claim 8 for testing a sample of body fluid.

It should be noted that embodiments and features described in the context of one of the aspects of the present invention also apply to the other aspects of the invention.

## Claims

1. Use of a handheld apparatus for testing a sample of body fluid; the apparatus comprising:
- a handheld test meter adapted for receiving a disposable electrochemical test unit; and
- a disposable electrochemical test unit configured for electrochemical sensing of a redox active substance;
wherein the disposable electrochemical test unit comprises a connector configured for connecting with the handheld test meter;
wherein an electronic identification tag is embedded in the disposable electrochemical test unit, the electronic identification tag comprising information on the type of disposable electrochemical test unit; wherein said electronic identification tag is a passive RFID tag;
wherein the handheld test meter is configured to read the electronic identification tag, and in response to its information on the type of disposable electrochemical test unit, configured to detect and process the electrochemical signal generated by the disposable electrochemical test unit during use to provide an indication to the user of the presence and/or amount of a redox active substance; **characterized in that** said handheld test meter comprises an insertion hole comprising a clamping section with an engagement member; wherein said connector comprises an engagement groove adapted to be engaged with the engagement member in the clamping section, wherein the electronic identification tag is embedded in the engagement groove, wherein the engagement member comprises an electronic identification tag reader.

2. Use according to claim 1, wherein said electronic identification tag is a passive Near Field Communication tag.

3. Use according to any one of the claims 1-2, wherein said RFID tag is configured to have a read range within the range of 0-20 mm.

4. Use of a disposable electrochemical test unit for testing a sample of body fluid; said disposable electrochemical test unit being configured for electrochemical sensing of a redox active substance;
wherein said disposable electrochemical test unit comprises a connector configured for connecting with a handheld test meter;
wherein an electronic identification tag is embedded in said disposable electrochemical test unit, said electronic identification tag comprising information on the type of disposable electrochemical test unit;
wherein said electronic identification tag is a passive RFID tag;
**characterized in that** said connector comprises an engagement groove adapted to be engaged with an engagement member in said clamping section of said handheld test meter, and wherein said electronic identification tag is embedded in said engagement groove.

5. Use according to claim 4, wherein said electronic identification tag is a passive Near Field Communication tag.

6. Use according to any one of the claims 4-5, wherein said RFID tag is configured to have a read range within the range of 0-20 mm.

7. Use according to any one of the claims 4-6, wherein said disposable electrochemical test unit comprises a receptacle for a sample of body fluid, and a diluent and/or solvent reservoir in liquid communication with the receptacle.

8. Use of a handheld test meter for testing a sample of body fluid; said handheld test meter being configured to read an electronic identification tag being a passive RFID tag;
wherein said passive RFID tag is embedded in a disposable electrochemical test unit, and in response to its information on the type of disposable electrochemical test unit, configured to detect and process the electrochemical signal generated by the disposable electrochemical test unit during use to provide an indication of presence and/or amount of a redox active substance to the user; **characterized in that** said handheld test meter comprises an insertion hole comprising a clamping section comprising an engagement member adapted for receiving an engagement groove of a disposable electrochemical test unit, and wherein said engagement member comprises an electronic identification tag reader adapted for reading the passive RFID tag.

## Patentansprüche

1. Verwendung einer tragbaren Vorrichtung zum Testen einer Körperflüssigkeitsprobe; wobei die Vorrichtung Folgendes umfasst:
- ein tragbares Testmessgerät, das zum Aufnehmen einer elektrochemischen Einwegtesteinheit angepasst ist; und
- eine elektrochemische Einwegtesteinheit, die zum elektrochemischen Erfassen einer redoxaktiven Substanz konfiguriert ist;
wobei die elektrochemische Einwegtesteinheit einen Anschluss umfasst, der zum Verbinden mit dem tragbaren Testmessgerät konfiguriert ist;
wobei ein elektronisches Identifikationsetikett in die elektrochemische Einwegtesteinheit eingebettet ist, wobei das elektronische Identifikationsetikett Informationen über den Typ der elektrochemischen Einwegtesteinheit umfasst; wobei das elektronische Identifikationsetikett ein passives RFID-Etikett ist;
wobei das tragbare Testmessgerät dazu konfiguriert ist, das elektronische Identifikationsetikett zu lesen, und als Reaktion auf seine Informationen über den Typ der elektrochemischen Einwegtesteinheit dazu konfiguriert ist, das elektrochemische Signal, das durch die elektrochemische Einwegtesteinheit während der Verwendung erzeugt wird, zu detektieren und zu verarbeiten, um dem Benutzer eine Angabe über das Vorhandensein und/oder die Menge einer redoxaktiven Substanz bereitzustellen; **dadurch gekennzeichnet, dass** das tragbare Testmessgerät ein Einführungsloch umfasst, das einen Klemmabschnitt mit einem Eingriffselement umfasst; wobei der Anschluss eine Eingriffsnut umfasst, die so angepasst ist, dass sie mit dem Eingriffselement in dem Klemmabschnitt in Eingriff gebracht werden kann, wobei das elektronische Identifikationsetikett in die Eingriffsnut eingebettet ist, wobei das Eingriffselement einen elektronischen Identifikationsetikett-Leser umfasst.

2. Verwendung nach Anspruch 1, wobei das elektronische Identifikationsetikett ein passives Nahfeld-Kommunikationsetikett ist.

3. Verwendung nach einem der Ansprüche 1-2, wobei der RFID-Tag dazu konfiguriert ist, einen Lesebereich im Bereich von 0-20 mm aufzuweisen.

4. Verwendung einer elektrochemischen Einwegtesteinheit zum Testen einer Körperflüssigkeitsprobe, wobei die elektrochemische Einwegtesteinheit zum elektrochemischen Erfassen einer redoxaktiven Substanz konfiguriert ist;
wobei die elektrochemische Einwegtesteinheit einen Anschluss umfasst, der zum Verbinden mit einem tragbaren Testmessgerät konfiguriert ist;
wobei ein elektronisches Identifikationsetikett in die elektrochemische Einwegtesteinheit eingebettet ist, wobei das elektronische Identifikationsetikett Informationen über den Typ der elektrochemischen Einwegtesteinheit umfasst;
wobei das elektronische Identifikationsetikett ein passives RFID-Etikett ist;
**dadurch gekennzeichnet, dass** der Anschluss eine Eingriffsnut umfasst, die angepasst ist, um mit einem Eingriffselement in dem Klemmabschnitt des tragbaren Testmessgeräts in Eingriff gebracht zu werden, und wobei das elektronische Identifikationsetikett in die Eingriffsnut eingebettet ist.

5. Verwendung nach Anspruch 4, wobei das elektronische Identifikationsetikett ein passives Nahfeld-Kommunikationsetikett ist.

6. Verwendung nach einem der Ansprüche 4-5, wobei der RFID-Tag dazu konfiguriert ist, einen Lesebereich im Bereich von 0-20 mm aufzuweisen.

7. Verwendung nach einem der Ansprüche 4-6, wobei die elektrochemische Einwegtesteinheit ein Gefäß für eine Körperflüssigkeitsprobe und ein Verdünnungsmittel- und/oder Lösungsmittelreservoir in Flüssigkeitsverbindung mit dem Gefäß umfasst.

8. Verwendung eines tragbaren Testmessgeräts zum Testen einer Körperflüssigkeitsprobe; wobei das tragbare Testmessgerät dazu konfiguriert ist, ein elektronisches Identifikationsetikett zu lesen, das ein passives RFID-Etikett ist;
wobei das passive RFID-Etikett in einer elektrochemischen Einwegtesteinheit eingebettet ist und als Reaktion auf seine Informationen über den Typ der elektrochemischen Einwegtesteinheit dazu konfiguriert ist, das durch die elektrochemische Einwegtesteinheit während der Verwendung erzeugte elektrochemische Signal zu detektieren und zu verarbeiten, um dem Benutzer eine Angabe des Vorhandenseins und/oder der Menge einer redoxaktiven Substanz bereitzustellen; **dadurch gekennzeichnet, dass** das tragbare Testmessgerät ein Einführungsloch umfasst, das einen Klemmabschnitt mit einem Eingriffselement umfasst, das zum Aufnehmen einer Eingriffsnut einer elektrochemischen Einwegtesteinheit angepasst ist, und wobei das Eingriffselement einen elektronischen Identifikationsetikett-Leser umfasst, der zum Lesen des passiven RFID-Etiketts angepasst ist.

## Revendications

1. Utilisation d'un appareil portatif pour tester un échantillon de fluide corporel ; l'appareil comprenant :
- un appareil de test portatif conçu pour recevoir une unité de test électrochimique jetable ; et
- une unité de test électrochimique jetable configurée pour la détection électrochimique d'une substance active redox ;
dans laquelle l'unité de test électrochimique jetable comprend un connecteur configuré pour se connecter au compteur de test portatif ;
dans laquelle une étiquette d'identification électronique est intégrée dans l'unité de test électrochimique jetable, l'étiquette d'identification électronique comprenant des informations sur le type d'unité de test électrochimique jetable ; dans laquelle ladite étiquette d'identification électronique est une étiquette RFID passive ;
dans laquelle l'appareil de test portatif est configuré pour lire l'étiquette d'identification électronique et, en réponse à ses informations sur le type d'unité de test électrochimique jetable, configuré pour détecter et traiter le signal électrochimique généré par l'unité de test électrochimique jetable pendant l'utilisation pour fournir une indication à l'utilisateur de la présence et/ou de la quantité d'une substance redox active ; **caractérisée en ce que** ledit appareil de test portatif comprend un trou d'insertion comprenant une section de serrage avec un élément d'engagement ; dans laquelle ledit connecteur comprend une rainure d'engagement conçue pour être en prise avec l'élément d'engagement dans la section de serrage, dans laquelle l'étiquette d'identification électronique est intégrée dans la rainure d'engagement, dans laquelle l'élément d'engagement comprend un lecteur d'étiquette d'identification électronique.

2. Utilisation selon la revendication 1, dans laquelle ladite étiquette d'identification électronique est une étiquette passive de communication en champ proche.

3. Utilisation selon l'une quelconque des revendications 1 et 2, dans laquelle ladite étiquette RFID est configurée pour avoir une plage de lecture comprise entre 0 et 20 mm.

4. Utilisation d'une unité de test électrochimique jetable pour tester un échantillon de fluide corporel ; ladite unité de test électrochimique jetable étant configurée pour la détection électrochimique d'une substance redox active ;
dans laquelle l'unité de test électrochimique jetable comprend un connecteur configuré pour se connecter au compteur de test portatif ;
dans laquelle une étiquette d'identification électronique est intégrée dans ladite unité de test électrochimique jetable, ladite étiquette d'identification électronique comprenant des informations sur le type d'unité de test électrochimique jetable ;
dans laquelle ladite étiquette d'identification électronique est une étiquette RFID passive ;
**caractérisée en ce que** ledit connecteur comprend une rainure d'engagement conçue pour être en prise avec un élément d'engagement dans ladite section de serrage dudit appareil de test portatif, et dans laquelle ladite étiquette d'identification électronique est intégrée dans ladite rainure d'engagement.

5. Utilisation selon la revendication 4, dans laquelle ladite étiquette d'identification électronique est une étiquette passive de communication en champ proche.

6. Utilisation selon l'une quelconque des revendications 1 et 2, dans laquelle ladite étiquette RFID est configurée pour avoir une plage de lecture comprise entre 0 et 20 mm.

7. Utilisation selon l'une quelconque des revendications 4 à 6, dans laquelle ladite unité de test électrochimique jetable comprend un réceptacle pour un échantillon de fluide corporel, et un réservoir de diluant et/ou de solvant en communication liquide avec le réceptacle.

8. Utilisation d'un appareil de test portatif pour tester un échantillon de fluide corporel ; ledit appareil de test portatif étant configuré pour lire une étiquette d'identification électronique qui est une étiquette RFID passive ;
dans laquelle ladite étiquette RFID passive est intégrée dans une unité de test électrochimique jetable et, en réponse à ses informations sur le type d'unité de test électrochimique jetable, configurée pour détecter et traiter le signal électrochimique généré par l'unité de test électrochimique jetable pendant l'utilisation pour fournir une indication de présence et/ou de quantité d'une substance active redox à l'utilisateur ; **caractérisée en ce que** ledit appareil de test portatif comprend un trou d'insertion comprenant une section de serrage comprenant un élément d'engagement adapté pour recevoir une rainure d'engagement d'une unité de test électrochimique jetable, et dans laquelle ledit élément d'engagement comprend un lecteur d'étiquette d'identification électronique adapté pour lire l'étiquette RFID passive.
